# EUROPEAN PATENT APPLICATION

(11) **EP 2 457 608 A1**
(43) Date of publication of application: **30.05.2012**
(21) Application number: 10192985.9
(22) Date of filing: 29.11.2010
(51) Int. Cl.: A61M 5/24, A61M 5/32

(54) **Medicated module and syringe**

(71) Applicant: Sanofi-Aventis Deutschland GmbH, 65929 Frankfurt (DE)
(72) Inventor: Mercer, David Richard, Warwickshire CV31 3BY (GB); Kouyoumjian, Garen, Warwickshire CV31 1HN (GB); Boyd, Malcolm Stanley, Warwickshire CV35 9PW (GB); De Sausmarez Lintell, Daniel Thomas, Warwickshire CV23 9EQ (GB)

(57) **Abstract**

A drug delivery system to deliver two or more medicaments operable through a syringe (5) and a single dispense interface (140). The device comprises a housing defining a primary reservoir (70), the primary reservoir containing a liquid. The device further includes a dosing mechanism (10) operably connected to the primary reservoir. Still further, the device includes a secondary reservoir for a medicament comprising at least one drug agent configured for fluid communication to the single dispense interface. Yet still further, the device includes a single dispense interface configured for fluid communication with the primary reservoir and the secondary reservoir. A single activation of the dosing mechanism causes liquid from the primary reservoir and a non-user settable dose of the medicament to be expelled through the single dispense interface.

## Description

### Field of the Present Patent Application

This invention relates to medical devices and methods of delivering at least two drug agents from separate reservoirs using a device having a dosing mechanism, such as a syringe, and a device having a single dispense interface. A single delivery procedure initiated by the user causes a non-user settable dose of a second drug agent and a variable dose of a first drug agent to be delivered to the patient. The drug agents may be available in two or more reservoirs, containers or packages, each containing independent (single drug compound) or pre-mixed (co-formulated multiple drug compounds) drug agents. Specifically, our invention concerns a medicated module where the user does not have to manually select or set the module to dispense the second drug agent. In one arrangement, where the medicated module comprises a needle guard, activation of the needle guard automatically causes the reservoir of secondary-reservoir medicament to engage with dispensing conduits to allow a dose of liquid (e.g., medicament) and a single fixed dose of the secondary-reservoir medicament to be available for injection. In another arrangement, where the medicated module does not comprise a needle guard, activation of a dosing mechanism causes a dose of a liquid and a single fixed dose of the secondary-reservoir medicament to be administered. The disclosed arrangements may be of particular benefit where the therapeutic response can be optimized for a specific target patient group, through control and definition of the therapeutic profile.

### Background

Certain disease states require treatment using one or more different medicaments. Some drug compounds need to be delivered in a specific relationship with each other in order to deliver the optimum therapeutic dose. This invention is of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus.

There are a number of potential problems that can arise when delivering two medicaments or active agents simultaneously. The two active agents may interact with each other during the long-term, shelf life storage of the formulation. Therefore, it is advantageous to store the active components separately and only combine them at the point of delivery, e.g. injection, needle-less injection, pumps, or inhalation. However, the process for combining the two agents needs to be simple and convenient for the user to perform reliably, repeatedly and safely.

A further problem is that the quantities and/or proportions of each active agent making up the combination therapy may need to be varied for each user or at different stages of their therapy. For example one or more active agents may require a titration period to gradually introduce a patient up to a "maintenance" dose. A further example would be if one active agent requires a non-adjustable fixed dose while the other is varied in response to a patient's symptoms or physical condition. This problem means that pre-mixed formulations of multiple active agents may not be suitable as these pre-mixed formulations would have a fixed ratio of the active components, which could not be varied by the healthcare professional or user.

Additional problems can arise where a multi-drug compound therapy is required, because many users cannot cope with having to use more than one drug delivery system or make the necessary accurate calculation of the required dose combination. This is especially true for users with dexterity or computational difficulties. In some circumstances it is also necessary to perform a priming procedure of the device and/or needle cannulae before dispensing the medicaments. Likewise, in some situations, it may be necessary to bypass one drug compound and to dispense only a single medicament from a separate reservoir.

Accordingly, there exists a strong need to provide devices and methods for the delivery of two or more medicaments in a single injection or delivery step that is simple for the user to perform. The present system and methods can overcome the above-mentioned problems by providing separate storage containers for two or more active drug agents that are then only combined and/or delivered to the patient during a single delivery procedure. Administering a dose of one medicament administers a fixed or determined dose of the secondary-reservoir medicament (*i.e.*, non-user settable). The disclosed systems and methods also give the opportunity for varying the quantity of one or both medicaments. For example, one fluid quantity can be varied by changing the properties of the injection device (*e.g.*, by varying the size or volume of the primary reservoir of the syringe or alternatively varying or changing the device's "fixed" dose of the secondary drug package). As just one example, the second fluid quantity can be changed by manufacturing a variety of secondary drug containing packages with each variant containing a different volume and/or concentration of the second active agent. The user or healthcare professional would then select the most appropriate secondary package or series or combination of series of different packages for a particular treatment regime.

One arrangement of our invention also provides a medicated module that automatically causes the secondary reservoir of medicament to come into fluid communication with the liquid upon activation of the needle guard. This eliminates the need for the user to manually set or adjust the medicated module after performing a priming step. Alternatively, a medicated module for use with either a prefilled or a non-prefilled syringe, may be provided without a needle guard. If the medicated module does not include a needle guard, the medicated module may require a manual switch over from priming mode to dispensing mode.

These and other advantages will become evident from the following more detailed description of the invention.

### SUMMARY

Our invention allows complex combinations of multiple drug compounds within a single drug delivery system. The invention allows the user to use a dosing mechanism of a drug delivery device (*e.g.*, a syringe) and dispense a primary-reservoir liquid and a secondary-reservoir medicament through a single dispense interface. This drug delivery device (*e.g.*, a syringe) controls the dosing mechanism of the device such that a predefined combination of the liquid and the medicament may be delivered when a dose of a primary-reservoir liquid is dispensed through the single dispense interface. The drug delivery system may include a primary reservoir holding a liquid (e.g., medicament, solution, solvent) and a secondary reservoir holding a medicament. The dose of the secondary-reservoir medicament is assumed to be small relative to the liquid , so the minimum dose of the liquid would be large enough to dispense substantially all of the secondary-reservoir medicament. The dose of the liquid may vary depending on what dose of liquid is desired or required. In an example, the liquid may be a medicament, so that the drug delivery system is configured to deliver a plurality of medicaments. In another example, the liquid may be a solution or solvent for flushing out the secondary-reservoir medicament, and thus the drug delivery system may in some cases deliver a single medicament.

In an example where the liquid is a medicament, by defining the therapeutic relationship between the individual drug compounds the proposed delivery devices and systems could help ensure that a patient/user receives the optimum therapeutic combination dose from a multi-drug compound device without the inherent risks associated with multiple inputs where the user has to calculate and set the correct dose combination every time they use the device. The medicaments can be fluids, defined herein as liquids or powders that are capable of flowing and that change shape at a steady rate when acted upon by a force tending to change its shape. Alternatively, one of the medicaments may be a solid that is carried, solubilized or otherwise dispensed with another fluid.

Accordingly, one specific aspect of particular benefit to users with dexterity or computational difficulties is that the single input and associated predefined therapeutic profile removes the need for a user to calculate their prescribed dose every time they use the device and the single input allows considerably easier setting and dispensing of the combined compounds.

In one preferred embodiment, a drug compound, such as insulin, contained within a primary device (*e.g.*, a syringe) could be used with a single use, user replaceable, medicated module that contains a single dose of a medicament and the single dispense interface (e.g., an injection needle). When connected to the primary device, the secondary-reservoir medicament is activated/delivered on dispense of the primary-reservoir liquid. Although our invention specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with our invention.

For example, the purposes of our invention the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂), Exendin-3, Liraglutide, or AVE0010 (H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH₂).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

In one preferred arrangement, Applicants' medicated module comprises a needle guard. In one such needle guard arrangement, there is provided a medicated module attachable to a drug delivery device that comprises an outer housing having a proximal end, a distal end, and an outer surface, where the proximal end preferably has a hub holding a double-ended needle and having a connector configured for attachment to a drug delivery device. There is a reservoir in a bypass housing within the outer housing that contains a medicament. The medicated module assembly of our invention may contain a needle guard that can reduce the risk of accidental needle sticks before and after use, reduce the anxiety of users suffering from needle phobia as well as preventing a user from using the device a subsequent time when the additional medicament has already been expelled.

The needle guard is preferably configured with a solid planar surface at its distal end that provides a large surface area that reduces the pressure exerted on the patient's skin, which allows the user to experience an apparent reduction in the force exerted against the skin. Preferably, the planar surface covers the entire distal end of the guard with the exception of a small needle pass through hole aligned axially with the needle. This pass through hole is preferably no more than 10 times greater in diameter than the outer diameter of the needle cannula. For example, with a needle outside diameter of 0.34mm, the pass through hole diameter D can be 3.4mm. Preferably, the pass through hole size should be large enough for the user to see that the device is primed (*i.e.*, a drop or more of medicament) while not being so large that it is still possible to reach the end of the needle with a finger (i.e. needle stick injuries before or after use). This difference between the hole size and cannula diameter is to allow for tolerances, to allow users to see the drop of liquid on the end of the cannula after priming (whether a transparent or non-transparent guard is used) while keeping the size small enough to prevent accidental needle stick injuries.

Further, the movable needle guard or shield is configured to move axially in both the distal and proximal directions when pressed against and removed from an injection site. When the needle assembly is removed or withdrawn from the patient, the guard is returned to post-use extended position. A drive tooth on the inside surface of the guard engages a stop on a track on the outer surface of the bypass housing to securely lock the guard from further substantial axial movement. Preferably a lock out boss on the outer surface of the bypass housing is configured to engage a lock out feature on the inner proximal surface of the outer housing at the completion of the injection to further lock the medicated module from any further use and prevent the needle(s) and/or bypass component from being able to substantially move within the system even if the guard is held in an axially locked condition. By "substantial" movement we do not mean the typical amount of "play" in a system, but instead we mean that the guard and/or distal needle do not move axially a distance that exposes the distal end of the cannula once it is locked out.

One goal of our invention is to eliminate the need to have the user manually operate the medicated module to change the state of the module from a priming state to a combination dose delivery state. Manually operated devices are sometimes not as intuitive as they could be and raise the risk of accidental misuse. One arrangement of Applicants' proposed medicated module solves this problem by utilizing energy stored within the module prior to delivery of the device to the user. The stored energy can come from a biasing member, such as a compressed spring. This stored energy is released during normal user operation of the module by actuating the mechanism and thus activating the state change from prime dose to combination dose. The mechanism aims to make this actuation imperceptible to the user, consequently making the user experience of the module very similar to that of a standard commercially available and accepted needle or safety needle (i.e., unpack module, attach to a drug delivery device, prime drug delivery device, administer a set dose along with single dose in the module). In this way, the module mechanism aims to reduce the risk of unintentional misuse and to improve usability by replicating an already accepted practice for similar injection methods.

As the module mechanism does not require the user to access external features on the module for the purposes of actuation, the number of components and subsequent module size can be reduced/optimized. These factors make the mechanism ideal for a single-use, high-volume manufacture, and disposable device application. Alternatively, as the actuation is driven by a single energy source, the system lends itself to a resettable actuation mechanism. One embodiment described below is the single use (non-resettable) version. The lower hub is preferably restrained rotationally with regard to the needle guard, but is free to move axially within the needle guard. The needle guard is restrained rotationally with regard to the outer housing, but is free to move axially, between defined constraints, within the outer housing.

The user pressing the distal face of the needle guard against the skin causes axial motion of the needle guard in the proximal direction. This axial motion of the guard causes a rotation of the bypass housing through the engagement and action of an inward-facing drive tooth on the guard as it travels in a drive track having one or more paths, which is located on the outer surface of the bypass housing. After sufficient axial travel of the needle guard, the rotation of the bypass housing brings stand-offs inside the outer housing and at the proximal ends of the lower hub into line with pockets located on the outer surface of the bypass housing. Alignment of the stand-offs with the pockets allows the bypass housing to move axially in the proximal direction and further into the outer housing. The lower hub containing a double-ended needle cannula moves axially further onto the bypass housing. Both of these movements occur due to the relaxation/release of the stored energy of the biasing member, preferably a spring that is pre-compressed during module assembly or manufacture, and constitute "triggering" of the actuation mechanism. It is this axial movement of the lower hub onto the bypass housing and the corresponding movement of the bypass housing further into the outer body that results in the double ended needles located in the outer body distal end and the lower hub piercing the medicated module, moving it from a state of priming to combination dose delivery.

Further axial movement of the needle guard is required in order to pierce the skin, this retraction of the needle guard temporarily re-compresses the biasing member creating additional stored energy. At a "commit" point, the proximal axial movement of the drive tooth passes a non-return feature in the track through further rotation of the bypass housing. In normal use, once the drug has been dispensed and the needle is removed from the skin, the needle guard is allowed to return axially in the distal direction under the relaxation of the biasing member as it releases its stored energy. At some point along its return travel, the drive tooth contacts a further ramped face in one of the paths of the track, resulting in yet further rotation of the bypass housing. At this point, the outer housing stand-off comes into contact with a ramp feature on the outer surface of the bypass housing. The combination of this feature with the ramp between the drive tooth and the bypass housing track results in further biasing of the bypass housing stop face into the needle guard drive tooth. The stop face features act as an axial locking pocket. The action of the combined biasing force means that any axial load in the proximal direction put on the needle guard will result in the tooth being stopped in this pocket, locking out the needle guard from further use or exposing the needle. Should the user remove the device from the skin without dispensing fluid, but after the "commit" point has been passed, the needle guard would return to an extended position and lock out as previously described.

In one embodiment, there is provided a medicated module assembly attachable to a drug delivery device, preferably a syringe, where the medicated module assembly comprises an outer housing having a proximal end and a distal end, where the proximal end has an upper hub holding a first double-ended needle cannula and a connector configured for attachment to a drug delivery device. The hub can be a separate part from the housing or integral, for example molded as part of the housing. The connector can be any connector design, such as threads, snap fits, bayonet, lure lock, or combination of these designs.

Two needle cannula are used, a distal cannula and a proximal cannula, with both cannulae preferably being doubled-ended for piercing a septum or seal near a distal end of the syringe and for piercing in injection site, such as a patient's skin. The distal needle is mounted in a lower hub and the proximal needle is mounted in the upper hub, each using any technique known to those skilled in the art, such as welding, gluing, friction fit, over-molding and the like. The medicated module assembly also contains a biasing member, preferably a compression spring. The biasing member is preferably in a pre-compressed state and positioned between the proximal inner face of the needle guard and the distal face of the lower hub. Although a preferred biasing member is a spring, any type of member that produces a biasing force will work.

The medicated module assembly automatically, once triggered, changes state from (1) a pre-use or priming state, where a small amount of liquid (e.g., medicament) flows in a bypass around the reservoir containing a single dose of the secondary-reservoir medicament, to (2) a ready-to-use or combination dose state, where both the upper cannula and lower cannula are in fluidic engagement with the fixed dose of the secondary-reservoir medicament within the module and where a set dose of the liquid (e.g., medicament) can be injected along with the non-settable single dose of secondary-reservoir medicament in the reservoir, and finally to (3) a locked out state, where the needle guard is prevented from substantial proximal movement. The outer housing preferably has a window or indicator that shows the various states of the module. The indicator can be a pip, knob, button, or the like that protrudes through the outer surface of the proximal end of the needle guard and visually shows the user whether the module is in the pre-use or ready-to-use state. It may also be a visual indicator, e.g. showing colors or symbols, or a tactile or audible indicator. Preferably, user noticeable indicia indicate both a pre-use priming position and a locked position of the guard after the medicated module assembly has been used to perform an injection. Inside the bypass housing there is a cavity that contains the capsule, which comprises the single dose of medicament in the reservoir. As the needle guard is retracted during an injection, the bypass housing is moved proximally along with the capsule positioned inside the cavity, thus decreasing the cavity volume. This allows the seals of the capsule to be pierced at its top and bottom by the needle cannula such that the medicament can be expelled from the reservoir during dose delivery. When connected to a drug delivery device containing a primary-reservoir liquid and prior to piercing the seals of the reservoir, the needle cannulae are only in fluid communication with the primary-reservoir liquid and a fluid flow path that bypasses the capsule. Preferably, a channel on the inside surface of the bypass housing is part of this fluid flow path and is used in the priming function of the drug delivery device.

As mentioned, the bypass housing preferably has one or more tracks located on the outside surface each having a set of first, second, third, and fourth paths. On the inner surface of the proximal end of the needle guard is one or more radial protrusions or drive teeth. As the guard first begins to retract, these protrusions travel in the first path causing the bypass housing to slightly rotate. As the guard continues to retract and then partially extend, the protrusions travel in the second and third paths. The protrusion moves to the fourth path and into a locking position when the guard is fully extended to its post-use position, which is preferably less extended than the starting position. The guard is rotationally constrained by the outer housing, preferably by the use of one or more spline features in the outer surface of the guard in cooperation with one or more followers or pips located at the distal end of the inner surface of the outer housing. The bypass housing is rotationally constrained when the protrusion is in the second path of the track. As the protrusion is moved axially in the proximal direction when the guard retracts, the protrusion moves from the second track to the third track causing the assembly to emit an audile sound and/or tactile feedback. This tells the user that the device has now been activated to lock upon extension of the guard in the distal direction.

A further aspect of the invention relates to a method of dispensing a fixed dose of one medicament and a variable dose of a primary-reservoir liquid (e.g., medicament) from separate reservoirs that involves the steps of first attaching a medicated module to a delivery device set in a pre-use or prime only state. The user can prime the dose delivery device using only the primary-reservoir liquid and bypassing the secondary-reservoir medicament. After priming the user begins the injection and the needle guard begins to retract and the module automatically changes to second state that allows a combination delivery of the two medicaments. Upon completion of the delivery procedure and retraction of the needle from the injection site, the extension of the needle guard automatically changes the module to a third state.

During dispense, substantially the entire amount of secondary-reservoir medicament has been expelled as well as a dose of the primary-reservoir liquid, through the single dispense interface. The capsule preferably contains a flow distributor to ensure that substantially all the single dose of secondary-reservoir medicament is forced out of the capsule by the liquid during an injection. The flow distributor can be a separate stand alone insert or pin. Alternatively the flow distributor and the capsule together can be manufactured or assembled as a one-piece component where the flow distributor is integral with the capsule. Such a unitary construction can be achieved utilizing, for example, design principles such as form fit, force fit or material fit, such as welding, gluing, or the like, or any combination thereof. The one-piece component may comprise one or more medicament flow channels, preferably one flow channel. The capsule and/or flow distributor can be constructed of any material that is compatible to the liquid and the secondary-reservoir medicament. Preferably the capsule and/or flow distributor can be made from compatible materials of construction that include, but are not limited to, COC (an amorphous polymer based on ethylene and norbonene, also referred to as cyclic olefin copolymer, ethylene copolymer, cyclic olefin polymer, or ethylene-norbornene copolymer); LCP (a liquid crystal polymer having an aramid chemical structure that includes linearly substituted aromatic rings linked by amide groups, and further can include partially crystalline aromatic polyesters based on p-hydroxybenzoic acid and related monomers and also highly aromatic polyesters); PBT (polybutylene terephthalate thermoplastic crystalline polymer or polyester); COP (a cyclic olefin polymer based on ring-opening polymerization of norbornene or norbornene-derivatives); HDPE (high density polyethylene); and SMMA (styrene methyl methacrylate copolymer based on methyl methacrylate and styrene). A preferred material is one that is typically used to manufacture septa or pistons (bungs) found in multi-dose medicament cartridges, however, any other material that is compatible with the drug could be used, e.g., glass, plastics or specific polymers, for example, TPE (thermo plastic elastomer); LSR (liquid silicone rubber); LDPE (low density polyethylene); and/or any kind of medical grade rubber, natural or synthetic. By "substantially all" we mean that at least about 80% of the secondary-reservoir medicament is expelled from the drug delivery device, preferably at least about 90% is expelled. In the third state, preferably the module is locked so as to prevent a second delivery or insertion by means of a locking mechanism as described previously.

The combination of compounds as discrete units or as a mixed unit is delivered to the body via an integral needle. This would provide a combination drug injection system that, from a user's perspective, would be achieved in a manner that very closely matches the currently available injection devices that use standard needles.

Another embodiment of our invention relates to a drug delivery system to deliver two or more medicaments through a single dose setter and a single dispense interface that comprises a housing containing a dosing mechanism comprising a plunger operably connected to a primary reservoir of medicament containing at least one drug agent. A dose button at one end of the plunger is also operably connected to the primary reservoir of medicament. This dose button can be any type of mechanism that triggers the delivery procedure, where driven mechanically or through a combination of electronics and mechanics. The button can move or be a touch sensitive virtual button, for example, a touch sensitive screen. Our system has a single dispense interface configured for fluid communication with the primary reservoir and with a secondary reservoir of medicament containing at least one drug agent. The drug dispense interface can be any type of outlet that allows the two or more medicaments to exit the system and be delivered to the patient. Types of interfaces include hollow needles, catheters, atomizers, pneumatic injectors, or needle-less injectors, mouth-pieces, nasal-applicators and the like interfaces.

The medicated module of our invention can be designed for use with any syringe with an appropriate compatible interface. However, it may be preferable to design the medicated module in such a way as to limit its use to one exclusive primary syringe (or family of syringes) through employment of dedicated/coded/exclusive features to prevent attachment of a non-appropriate medicated module to a non-matching syringe. In some situations it may be beneficial to ensure that the medicated module is exclusive to one syringe while also permitting the attachment of a standard drug dispense interface to the syringe. This would allow the user to deliver a combined therapy when the module is attached, but would also allow delivery of the primary-reservoir liquid independently through a standard drug dispense interface in situations, such as, but not limited to, dose splitting or top-up of the primary compound.

A particular benefit of the presently disclosed devices and systems is that the medicated module makes it possible to tailor dose regimes when required, especially where a titration period is necessary for a particular drug. The medicated module could be supplied in a number of titration levels with obvious differentiation features such as, but not limited to, aesthetic design of features or graphics, numbering etc, so that a patient could be instructed to use the supplied medicated module in a specific order to facilitate titration. Alternatively, the prescribing physician may provide the patient with a number of "level one" titration medicated modules and then when these were finished, the physician could then prescribe the next level. A key advantage of this titration program is that the primary device remains constant throughout.

In a preferred embodiment of our invention, the primary drug delivery device is used more than once and therefore is multi-use; however, the drug delivery device may also be a single use disposable device, such as a disposable syringe. It is also possible to have a suite of different medicated modules for various conditions that could be prescribed as one-off extra medication to patients already using a standard drug delivery device. Should the patient attempt to reuse a previously used medicated module, a presently disclosed embodiment includes a locking needle guard that is activated after a first predefined travel/retraction of the guard/insertion of the needle. The locked needle guard would alert the patient to this situation and the inability to use the module for a second time. Visual warnings (e.g. change in color and/or warning text/indicia within an indication window on the module once insertion and/or fluid flow has occurred) can also be used. Additionally, tactile feedback (presence or absence of tactile features on the outer surface of the module hub following use) could be used as well.

A further feature of our invention is that both medicaments are delivered via one injection needle and in one injection step. This offers a convenient benefit to the user in terms of reduced user steps compared to administering two separate injections. This convenience benefit may also result in improved compliance with the prescribed therapy, particularly for users who find injections unpleasant or who have computational or dexterity difficulties.

Our invention also covers a method of delivering two medicaments stored in separate primary packages. The medicaments may both be liquid, or alternatively one or more of the medicaments may be a powder, suspension or slurry. In one embodiment the medicated module could be filled with a powdered medicament that is either dissolved or entrained in the liquid as it is injected through the medicated module.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates one arrangement of a syringe that can be used with the present invention;
Figure 2 illustrates one arrangement of a distal end of a drug delivery device, such as the distal end of the syringe illustrated in Figure 1;
Figure 3 illustrates an embodiment of a medicated module of the present invention, where the medicated module is separated from a primary reservoir, such as the primary reservoir of the syringe illustrated in Figure 1;
Figure 4 illustrates the medicated module illustrated in Figure 2 attached to a distal end of a drug delivery device, such as the syringe illustrated in Figure 1;
Figure 5 illustrates an exploded distal perspective view of all the components (except the medicated capsule) of the medicated module illustrated in Figure 2;
Figure 6 illustrates an exploded proximal perspective view of all the components (except the medicated capsule) of the medicated module illustrated in Figure 2;
Figure 7 is a perspective view of the capsule containing the reservoir of the embodiment of Figure 2;
Figure 8 illustrates a proximal perspective view of the outer housing of the embodiment of Figure 2;
Figure 9 is a sectioned view of the embodiment of the medicated module shown in

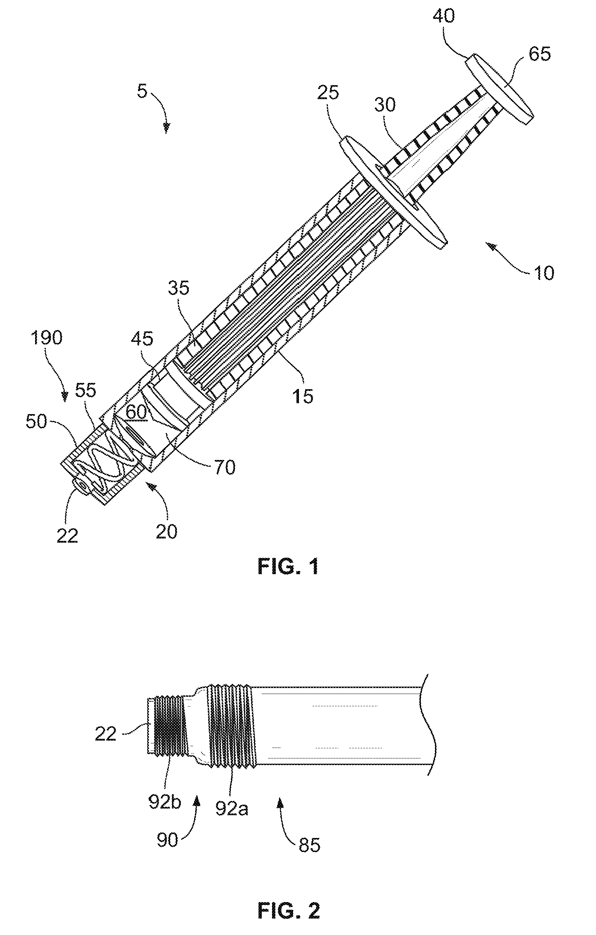

Figure 2 orientated in the bypass configuration; and

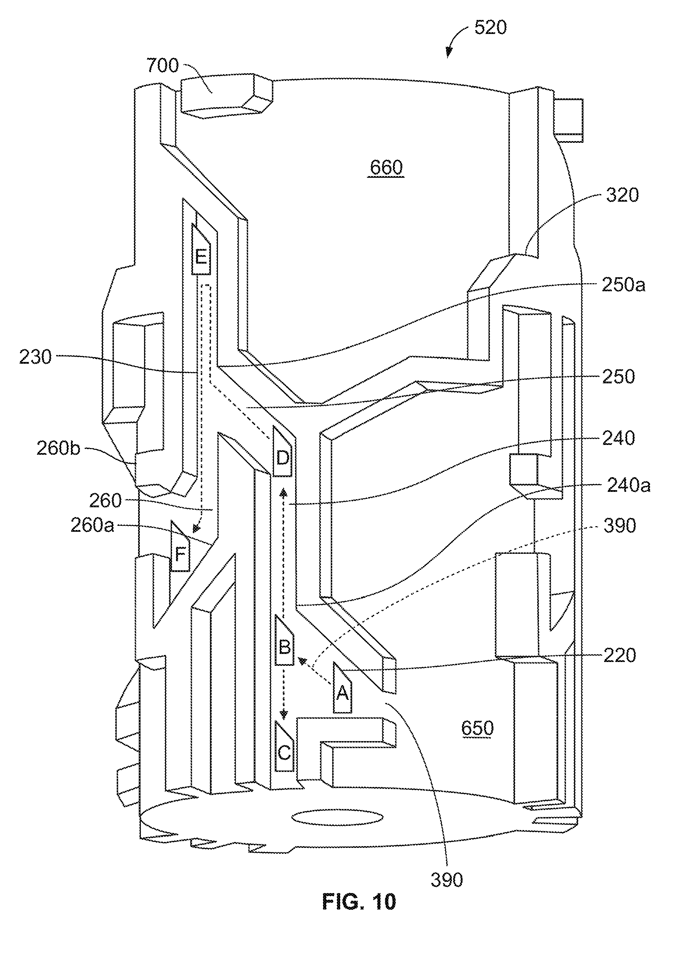

Figure 10 is a close-up perspective view of the bypass housing of the embodiment of the medicated module shown in Figure 2 to illustrate the positions of the drive tooth during use.

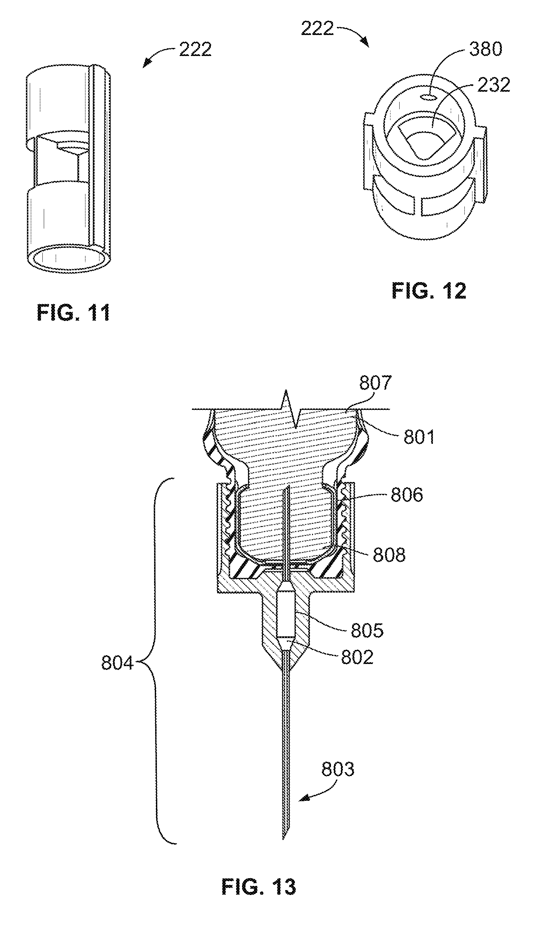

Figure 11 illustrates one possible embodiment of a reservoir for use with the medicated module illustrated in Figure 2.
Figure 12 illustrates one possible embodiment of a flow distributor for use with the medicated module illustrated in Figure 2.
Figure 13 illustrates an alternative embodiment of Applicants' medicated module comprising two needles connected to a secondary reservoir attached to a drug delivery device, such as the drug delivery device illustrated in Figure 1.

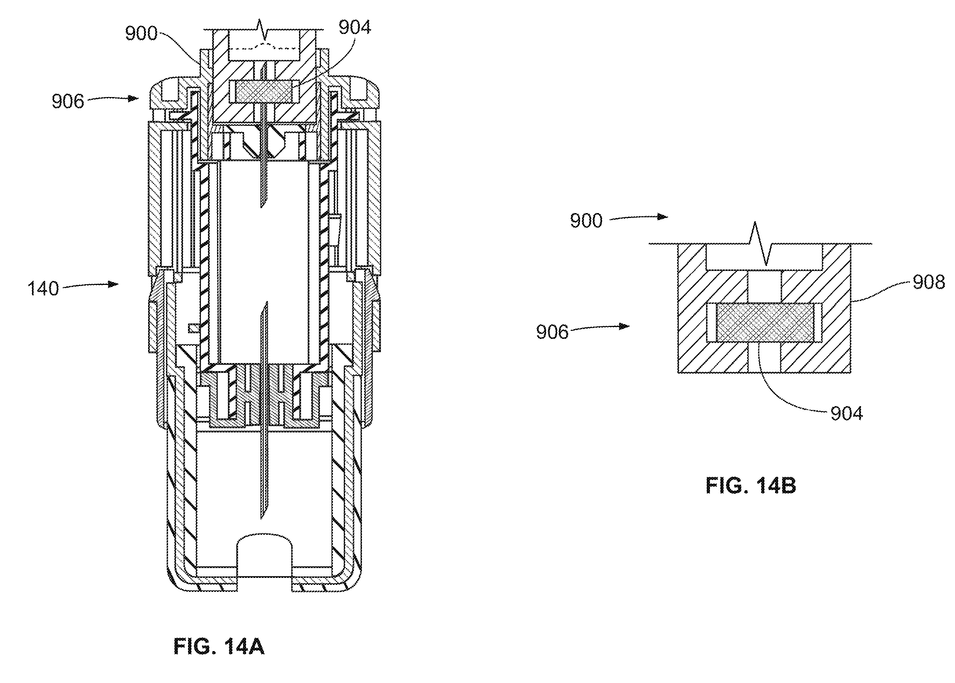

Figure 14a illustrates a distal end of a syringe attached to a medicated module.
Figure 14b illustrates a close-up view of the distal end of the syringe of Figure 14a.

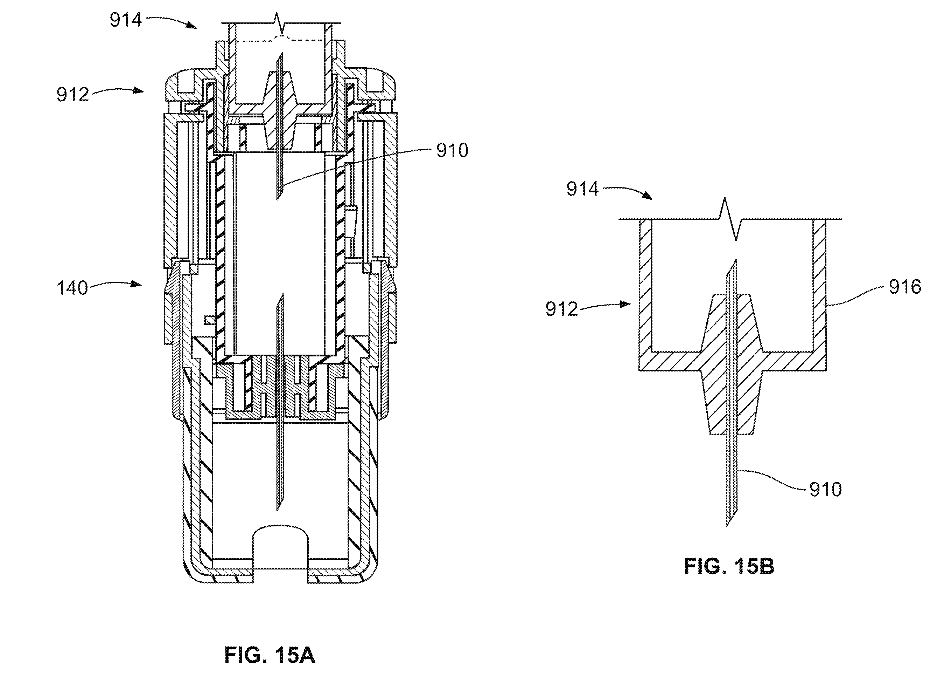

Figure 15a illustrates a distal end of a syringe attached to a medicated module.
Figure 15b illustrates a close-up view of the distal end of the syringe of Figure 15a.

### DETAILED DESCRIPTION

The present invention administers a fixed predetermined dose of a drug compound (i.e., medicament) and a variable dose of a liquid (e.g., a medicament, solution, solvent., etc) primary or first drug compound through a single output or drug dispense interface. By the user administering a dose of the liquid, the user automatically determines the fixed dose of the secondary-reservoir medicament, which preferably is a single dose contained in a capsule or reservoir having an integral flow distributor. In a preferred embodiment the drug dispense interface is a needle cannula (hollow needle).

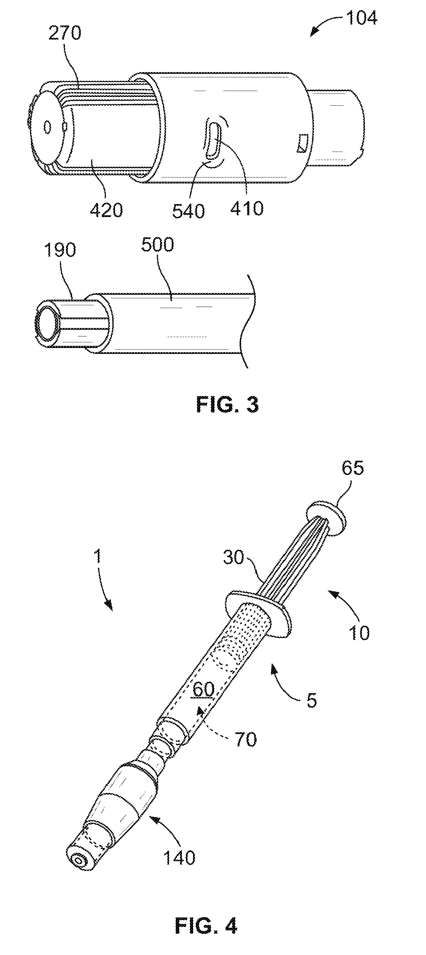

Figure 1 illustrates one example of a drug delivery device 5 that the medicated module 140 (see Figures 3 - 13) of our invention can be attached to the connection means 190 on a distal end 20 of the syringe body 15. Each medicated module is preferably self-contained and provided as a sealed and sterile disposable module that has an attachment means compatible to the attachment means 190 at the distal end 20 of device 5. Although not shown, the medicated module 140 could be supplied by a manufacturer in a protective and sterile container, where the user would peel or rip open a seal or the container itself to gain access to the sterile medicated module. In some instances it might be desirable to provide two or more seals for each end of the medicated module.

In one preferred arrangement, and as illustrated in Figure 1, the drug delivery device 5 comprises a syringe. Such a syringe may comprise a prefilled syringe. By prefilled syringe, it is meant that a user of the syringe receives the device with the liquid (e.g., medicament) already provided within the primary reservoir of the device. Alternatively, the syringe may comprise a reusable syringe: one that can be repeatedly filled from a vial just before use.

In one arrangement, the syringe comprises a sterile syringe device 5. As illustrated, the syringe comprises a syringe body or housing 15 that preferably has a generally tubular configuration. The syringe body 15 helps to define a primary reservoir 70 that contains a liquid (e.g., medicament) 60. The syringe body 15 extends from a distal end 20 to a proximal end 25. At the proximal end 25 of the syringe body 15, a dosing mechanism 10 is provided. This dosing mechanism 10 allows a user to administer a dose of the liquid 60 contained with the primary reservoir 70. In this arrangement, the dosing mechanism 10 comprises a plunger mechanism 30. This plunger mechanism 30 is positioned within an interior of the syringe housing 15 and positioned so that it can move in either the distal or proximal direction.

The plunger mechanism 70 comprises a piston 45 at the distal end 35 of the plunger mechanism. This piston frictionally engages an inner surface of the syringe body 15 and acts within the interior chamber of the housing 15 to define the primary reservoir 70 therein in which fluid medium is contained. The distal end 20 of the syringe body 15 is configured to have secured thereon a medicated module (such as that shown in Figure 2) for use in providing fluid flow communication between the primary reservoir 70 and that environment from which the liquid 60 is to be administered. A septum may be provided at the distal end of the device. Fluid communication between the syringe and the medicated module may be achieved in various ways. In some cases, the medicated module may have a proximal needle that may pierce the septum of the syringe. For example, as shown in Figure 14a, a septum 904 is provided at the distal end 906 of the syringe 900. Generally, this septum 904 functions in the same manner as the septum of a cartridge. The medicated module, such as medicated module 140, may then be attached to the distal end 906 of the syringe 900.

In this example, to fill the syringe 900 from a vial (e.g., for a non-pre-filled syringe), it would be necessary to attach a needle in order to pierce the septum 904 and allow transfer of fluid to the syringe 900. In other words, the needle to facilitate fluid transfer to the syringe would have to be external to the syringe.

The septum 904 may be provided to the syringe 900 in a variety of ways, including but not limited to insert-moulding, over-moulding, or some other similar method. Figure 14b provides a detailed view of the septum 904 provided at the distal end 906 of the syringe 900. This is one example configuration of the design of the distal end. In addition, exclusive attachment means for attachment to a medicated module may be provided on the external surface 908 of the syringe.

In other examples, the syringe itself may have a distal needle. For example, as shown in Figures 15a-b, a needle, such as needle 910, may be fixed to the distal end 912 of the syringe 914. In this example, the needle 910 serves to replace the proximal needle in the medicated module 140, such as proximal needle 150. This needle 150 would instead be replaced by a hole or pathway that allows the syringe needle 910 to pass through and communicate with the primary pack reservoir of the medicated module 140. The syringe needle 910 may be connected to the distal end of the syringe in a variety of ways, including, but not limited to, being bonded, over-moulded, or welded into the syringe distal end. Figure 15b provides a detailed view of the septum the distal end 912 of the syringe 914. This is one example configuration. In addition, exclusive attachment means for attachment to a medicated module may be provided on the external surface 916 of the syringe.

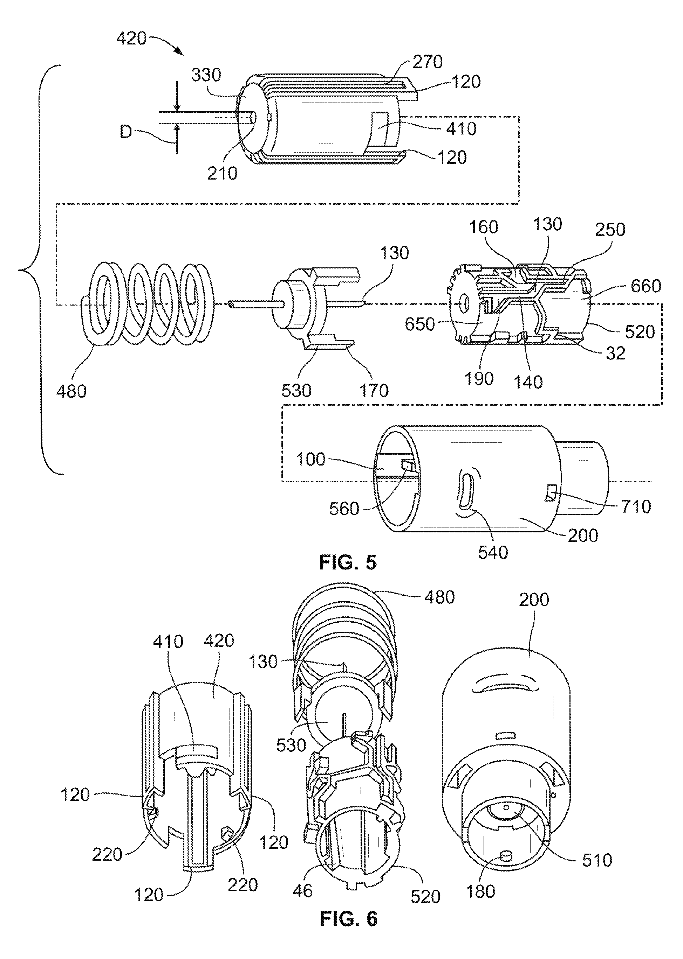

Returning to Figure 1, the syringe 5 comprises a coupling mechanism 190 for releasably coupling a medicated module, such as the medicated module 140 illustrated in Figure 2. As just one example, in one arrangement, the syringe delivery device 5 may include a coupling mechanism 90 in the form of a threaded section 50. This threaded section 50 of the syringe 5 threadably couples a corresponding attachment means or connector 180 on the medicated module 140. (See, *e.g.*, Figure 5) Alternatively, in another drug delivery device arrangement, other known coupling mechanism 190 can be used to attach the medicated module to the distal end of the drug delivery device. This could include types of permanent and/or removable connection means, such as threads, snap locks, snap fits, luer locks, bayonet, snap rings, keyed slots, and combinations of such connections.

In some situations, however, it may be also beneficial to ensure that the medicated module 140 is exclusive to one syringe while also permitting the attachment of a standard needle, such as a standard double ended needle assembly that is generally known to those skilled in the art. One advantage of such an arrangement is that such a situation would allow the user to deliver a combined therapy when the medicated needle 140 is attached to the drug delivery device but would also allow the user to deliver a primary-reservoir compound independently through a standard needle in situations such as, but not limited to, dose splitting or top-up of the primary-reservoir compound or where the secondary-reservoir compound was not required at all. In the latter situation, a prefilled syringe could be used on its own to deliver the liquid (e.g., medicament). Then, if and when required, the medicated module could be used to deliver a secondary-reservoir medicament without the need for two injections. The medicated module could have an exclusive interface such that it can only be used with one such dedicated pre-filled syringe for reasons such as safety. A range of medicated modules could contain a variety of medicaments for various situations/scenarios all of which could be delivered in combination with the medicament from the syringe (primary device). It is also possible that the syringe contains only a buffer solution or dilutant to dispense a concentrated medicament from the medicated module.

One such exclusive attachment means for achieving the partial exclusive attachment is illustrated in Figure 2. For example, Figure 2 illustrates a distal end 85 of a drug delivery device 80, such as the syringe 5 illustrated in Figure 1. As illustrated, the distal end 85 comprises a coupling mechanism 90. This coupling mechanism comprises a first attachment means 92a and a second attachment means 92b. In this illustrated arrangement, both the first and second attachment means 92a, 92b are shown in the form of a first and a second threaded sections. In this example, the first threaded section 92a permits the attachment of a medicated module, such as the medicated module illustrated in Figure 3. The second threaded section 92b permits the attachment of a standard needle double ended needle assembly. Although only showing threaded interfaces, other coupling mechanisms could also be used for the standard needle section and the means of attaching the medicated needle is not limited to just screw threads. With such a coupling mechanism 90, a medicated module could be releasably coupled to the syringe. For example, Figure 4 illustrates a drug delivery system 1 comprising a medicated module 140 coupled to a syringe, such as the syringe 5 illustrated in Figure 1. However, in another example, a syringe may be a single-use syringe. In such a case, the attachment interface could be such that once the medicated module was attached, the syringe and medicated module may not be decoupled, thereby fixing the two elements together for disposal.

Figures 3-10 illustrate an alternative embodiment of a medicated module comprising a needle guard. For example, Figs. 3, and 4 illustrate an alternative attachment means 190 of a drug delivery device, such as the syringe illustrated in Figure 1. As illustrated, this attachment means 190 comprises a unique bayonet type connection that is keyed specifically to a corresponding female bayonet type connection 180 on hub 510 of medicated module 140. The embodiments shown in Figure 3, 5, 6, and 9 have the benefit of the secondary-reservoir medicament as a single dose being contained entirely within capsule 310, and specifically in reservoir 222, hence minimizing the risk of material incompatibility between the secondary-reservoir medicament and the materials used in the construction of the medicated module 140, specifically housing 200, inner housing 520, or any of the other parts used in the construction of the medicated module.

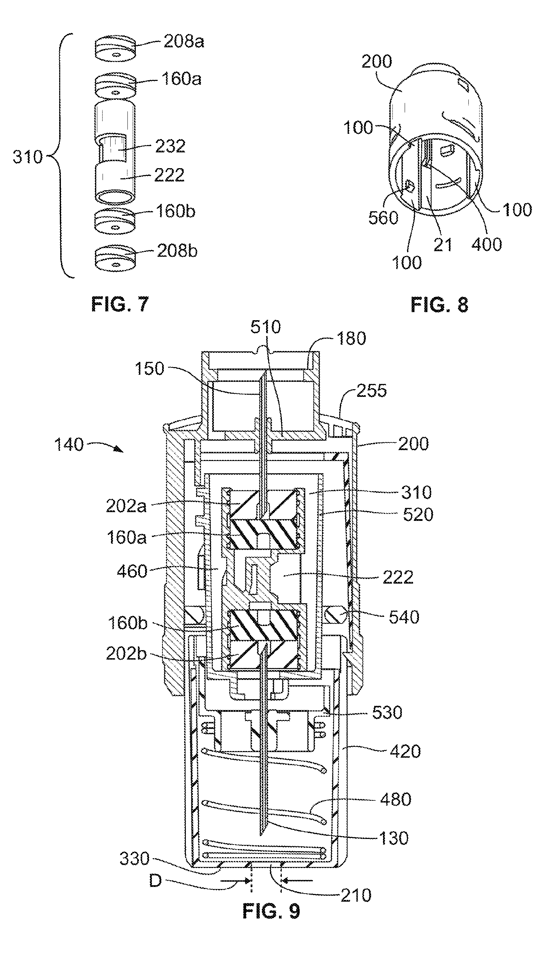

To minimize the residual volume of the secondary-reservoir medicament, caused by recirculation and/or stagnant zones, that might remain in capsule 310 at the end of the dispense operation, it is preferable to have a flow distributor 232 as an integral part of reservoir 222 (see Figure 7). The reservoir 222 containing the single dose of the secondary-reservoir medicament can be sealed with septa 160a and 160b, which are fixed to the capsule using keepers or plugs 202a and 202b. Preferably the keepers have fluid channels that are in fluid communication with needles 130 and 150 and with bypass 460, which is preferably part of the inside surface of bypass housing 520. Together this fluid path allows priming of the drug delivery device before injection. Preferably the reservoir, flow distributor, keepers, and bypass can be made from materials that are compatible with the liquid (e.g., medicament). Examples of compatible materials of construction include, but are not limited to, COC (an amorphous polymer based on ethylene and norbonene, also referred to as cyclic olefin copolymer, ethylene copolymer, cyclic olefin polymer, or ethylene-norbornene copolymer); LCP (a liquid crystal polymer having an aramid chemical structure that includes linearly substituted aromatic rings linked by amide groups, and further can include partially crystalline aromatic polyesters based on p-hydroxybenzoic acid and related monomers and also highly aromatic polyesters); PBT (polybutylene terephthalate thermoplastic crystalline polymer or polyester); COP (a cyclic olefin polymer based on ring-opening polymerization of norbornene or norbornene-derivatives); HDPE (high density polyethylene); and SMMA (styrene methyl methacrylate copolymer based on methyl methacrylate and styrene). The needle pierceable septa, bungs, and/or seals that are used with both the capsule and the liquid (e.g., medicament) cartridge can be manufactured using TPE (thermo plastic elastomer); LSR (liquid silicone rubber); LDPE (low density polyethylene); and/or any kind of medical grade rubber, natural or synthetic.

The design of flow distributor 232 should ensure that at least about 80% of the secondary-reservoir medicament is expelled from reservoir 222 through the distal end of needle 130. Most preferably at least about 90% should be expelled. Ideally, displacement of the primary-reservoir liquid of a primary reservoir contained within a primary device, such as the drug delivery device 5, and through the capsule 310 will displace the single dose of the secondary-reservoir medicament stored in reservoir 222 without substantial mixing of the two medicaments.

Attachment of the medicated module 140 to the delivery device 5 causes proximal needle 150 to penetrate a septum 22 sealing the distal end 20 of the syringe body 15, and hence the primary reservoir 70 containing the liquid (e.g., medicament) of the drug delivery device 5. For example, Figure 4 illustrates the medicated module 140 attached to the syringe 5 where a proximal needle of the medicated module 140 pierces a septum of the syringe 5 so as to reside in fluid communication with the medicament 60 contained within the primary reservoir 70 of the syringe 5.

Once needle 150 of the medicated module 140 has passed through the septum 22 of the primary reservoir 70, fluid connection is made between the first or liquid 60 and the needle 150. At this point, the system can be primed by pushing on the proximal end 40 of the plunger 30 of the dosing mechanism 10 so that the plunger moves in the distal direction. As the piston 45 near the distal end of the plunger acts on the primary-reservoir liquid 60 contained in the primary reservoir 70, a small number of units will be expelled from the medicated module.

Once the device 5 is primed, then activation of the needle guard 420 allows dispense of the medicaments by subcutaneously injecting the medicaments via further activation of plunger 30 on device 5. The plunger 30 of the dosing mechanism 10 can comprise any triggering mechanism that causes the dose of the primary-reservoir liquid to move towards the distal end 20 of the device 5. In a preferred embodiment, the dosing button 65 is operably connected to a plunger that engages a piston in the primary reservoir of the medicament.

One embodiment of the medicated module 140 of our invention is illustrated in Figures 3 and 9. In these embodiments the medicated module 140 contains a capsule 310 comprising a reservoir 222, two keepers 202a and 202b, and two seals 160a and 160b. Reservoir 222 contains a fixed single dose of a secondary-reservoir medicament. In some cases this secondary-reservoir medicament may be a mixture of two or more drug agents that can be the same or different from the primary-reservoir liquid in the drug delivery device 5. Preferably the capsule is permanently fixed within the medicated module, however, in some cases it may be preferred to design the module such that the capsule can be removed when empty and replaced with a new capsule.

In the embodiments shown in Figures 7 and 9, capsule 310 has ends that are sealed with pierceable membranes or septa 160a and 160b that provide a hermetically sealed and sterile reservoir 222 for the secondary-reservoir medicament. A primary or proximal engagement needle 150 can be fixed in hub 510 connected to the proximal end of housing 200 of the module and configured to engage capsule 310 when needle guard is moving in the proximal direction during injection. The outlet, or distal needle 130, is preferably mounted in lower hub 530 and initially protrudes into lower keeper 202b. The proximal end of needle 130 pierces the lower septum 160b when the bypass housing 520 rotates and is moved proximally by the force exerted by needle guard 420 and spring 480 during injection.

When first attached to the syringe, the medicated module 140 is set at a pre-use or starting position. Preferably, indicator 410 shows through window 540 to inform the user of the pre-use condition of the medicated module. The indicator is preferably a color stripe or band on the outer surface of the proximal end of guard 420 (see Figure 5) visible through an aperture in the outer body. The needle guard 420 is slidably engaged with inner surface of outer housing 200 by engagement of arms 120 and channels 100. Retention snaps 560 prevent the guard from disengaging the outer housing at its fully extended position. Housing 200 partially defines an internal cavity 21 that holds bypass housing 520, which contains capsule 310. A portion of the proximal end of housing 200 defines an upper hub 510 that holds needle 150. Optionally, as illustrated in Figure 9, a shoulder cap 255 may be added to the proximal outer surface of outer housing 200. This shoulder cap can be configured to serve as indicia to identify to a user the type/strength of medicament contained in the module. The indicia can be tactile, textual, color, taste or smell.

Figure 9 shows a cutaway or cross-sectioned view of the medicated module set in a pre-use or starting state where needles 130 and 150 are not piercing septa 160a and 160b. In this position, the bypass housing 520 is at its most extended position and needles 130 and 150 are not in fluid communication with medicament contained in capsule 310. The capsule is supported by bypass housing 520. In this neutral or suspended state of capsule 310, liquid 60 from the primary reservoir 70 in syringe body 15 of syringe 5 can flow through needle 150 into keeper 202a, through bypass 460 and into keeper 202b, and eventually out through needle 130. This flow configuration allows a user to perform a priming step or procedure by administering a small dose of the liquid using the dosing mechanism 10 the drug delivery device 5.

The compression spring 480 is positioned between the distal end of bypass housing 520 and the inner proximal face of guard 420 to bias the guard 420 into an extended (guarded) position as illustrated in Figure 9. Upon assembly, spring 480 is purposely compressed to supply a proximally directed biasing force against lower hub 530. This pre-compression of spring 480 is possible because the lower hub 530 and the bypass housing 520 are prevented from moving in an axial proximal direction by radial stand off 400 located on the inside surface of the outer housing (Figure 8) that engage with an upper stand off pocket 660 and legs 270 of lower hub 530 engaging lower stand off pocket 650. The combination of these stand-offs/legs and pockets prevent the lower hub and upper hub needles from piercing into the centre of the capsule until the device is triggered as previously described.

The proximal inside surface of guard 420 has one or more inwardly protruding features, drive teeth, pips, or like structures 220 that run in one or more tracks 230 or guide ways formed in the outer surface of bypass housing 520. As shown in Figure 10, track 230 can be described as four paths, 390, 240, 250, and 260, that have a specific geometry such that after a single use of the medicated module 4 the drive tooth 220 is blocked from further axial movement and the guard (and device) is "locked" in a guarded position where the distal end of the needle is completely and safely covered by guard 420.

One unique feature of our medicated module assembly is the user feedback that is given when the assembly is used. In particular, the assembly could emit an audible and/or tactile "click" to indicate to the user that they have firstly triggered the device and secondly reached the "commit" point such that the needle guard will lock safely out upon completion of the injection/removal of the guard from the injection site. This audible and/or tactile feature could work as follows. As mentioned, the needle guard 420 is rotationally constrained by outer housing 200 and has one or more drive teeth 220 that are initially in path 290 of track 230 on bypass housing 520. As the guard is moved proximally, the spring 480 is further compressed exerting additional force in the proximal direction on lower hub 530, which is initially constrained axially by the lower stand off pocket 650 engaged with legs 270. Likewise, the bypass housing 520 is constrained from moving proximally by and upper stand off pocket stop (not shown) engaged with stand off 400 on the inner surface of outer housing 200. The drive teeth 220 travel in path 390 causing the bypass housing to rotate slightly. This rotation will disengage the upper stand off 400 from upper standoff pocket stop (not shown), allows the drive teeth to enter path 240, and unblocks legs 170 from lower standoff pocket the combination of which allows the bypass housing to move proximally carrying with it capsule 310, where it then can engage needles 130 and 150. As the guard continues to move proximally, the drive teeth move from path 240 passed transition point 240a into path 250 causing further rotation of the bypass housing. As this rotation is completed the drive teeth transition to path 230, potentially emitting an audile "click" sound, as well as a tactile feel, to the user. This transition past point 250a (and the corresponding point directly below it on the track) constitute the "commit" point and as such, once it has been reached, the needle guard 420 will "lock out" when it extends upon removal of the device from the injection site.

As mentioned, the distal end of the guard 420 has a planar surface 330 that provides an added measure of safety and reduces the pressure exerted by the guard on the injection site during an injection with our needle assembly. Because the planar surface 330 substantially covers access to needle 130 a user is prevented from gaining access to the distal tip of the needle after the assembly is in the locked position. Preferably, the diameter D of needle pass through hole 210 in the planar surface is no more than 10 times that of the outer diameter of needle cannula 130.

The outer proximal surface of the needle guard 420 preferably has indicia 410 that are preferably at least two different color stripes or bands, each of which is sequentially visible through the opening or window 540 in outer housing 200. One color could designate the pre-use or prime state of the module and the other color would indicate that the module is in finished or locked state, another color could be used to denote the transition through the trigger or "commit" point in case a user stops injection after trigger point but before "commit" point. For example, a green color could be the pre-use position and a band of red color could be used to indicate that the module has been used and is locked and an orange color could indicate that the device has been triggered but not locked out. Alternatively, graphics, symbols or text could be used in place of color to provide this visual information/feedback. Alternatively these colors could be displayed using the rotation of the bypass cavity and printed on or embedded into the bypass housing. They could be visible through the aperture by ensuring that the needle guard is made form a transparent material.

Figure 10 illustrates the travel of drive teeth 220 in one or more tracks 230 as illustrated by directional arrow 390. Drive tooth 220 begins at position A and through axial movement of the needle guard biases the bypass housing rotationally until it moves past the transition point 240a and arrives at position B. Once the drive tooth reaches position B the bypass housing and lower needle hub move proximally causing the capsule 310 to engage needles 130 and 150, and the drive tooth moves relatively to position C (this is termed as the triggering of the device) and it is the bypass housing/lower hub moving proximally under the release of stored energy that results in the effective position of the needle guard drive tooth being position C. It is important to note that the needle guard does not move under the action of the release stored energy, it is just the needle hub and the bypass housing that move relatively away from the needle guard at the point of triggering, hence the drive tooth moves from position B to position C. As the needle guard continues to retract, drive tooth 220 moves proximally in path 240 to position D, where it exerts a rotational bias on the bypass housing 520 causing it to rotate again until tooth 220 passes the transition 250a (commit point) into path 260. The drive tooth then moves proximally until position E is reached. At this point, the needle guard 420 is fully retracted and the full available insertable length of the needle is exposed. Once the user removes the guard from contact with the skin, the guard begins to extend as a result of the distal biasing force exerted by spring 480 on the inner proximal surface of the guard.

The utilization of the stored energy spring to act both as a trigger/piercing spring and also, once extended post triggering, as the needle guard spring, is a unique aspect of this design. It negates the need to use two separate springs for these separate functions by locating the spring in a position such that it can fulfill both roles. Initially, for example during assembly or manufacture of the medicated module, the biasing member is compressed exerting a force on the lower hub/bypass housing in preparation for triggering. Once triggered it extends proximally where upon it can then be compressed from the distal end as the needle guard retracts against it. This secondary compression provides the force to push the needle guard back to the extended and locked position as it is removed from the injection site. As the guard moves to its fully extended post-use position, which preferably is less extended than the starting position, the drive tooth 220 moves distally in path 250 until it reaches transition point 260a, where it then rotationally biases the bypass housing 520 to rotate yet again until tooth 220 enters path 260 and arrives at position F. This last rotation of bypass housing 520 causes lock out boss 700 to engage a lock out feature (not shown). This prevents any further rotational or axial movement of the bypass housing. The needle guard is prevented from further substantial axial movement, as defined earlier, by engagement of the drive tooth with axial stop 260b. It is within the scope of our invention that a number of tooth arrangements and/or profiles could be used to fulfill the required function described above, e.g., simple equal tooth profiles or more complex multi-angled profiles. The particular profile being dependent upon the required point of commit and rotation of the bypass housing. It is also within the scope of our invention that a similar axial/rotational locking of the lower needle hub to the bypass housing as of the bypass housing to the outer housing, could be integrated to prevent axial movement of the needle post-triggering and post-lock out.

In any of the above described embodiments of our invention the secondary-reservoir medicament may be either in a powdered solid state, any fluid state contained within the secondary reservoir or capsule, or coated to the inside surface of the drug dispense interface. The greater concentration of the solid form of the medicament has the benefit of occupying a smaller volume than the liquid having lower concentration. This in turn reduces the ullage of the medicated module. An additional benefit is that the solid form of the secondary-reservoir medicament is potentially more straightforward to seal in the secondary reservoir than a liquid form of the medicament. The device would be used in the same manner as an exemplary embodiment with the secondary-reservoir medicament being dissolved by the primary-reservoir liquid during dispense.

To minimize diffusion of the secondary-reservoir medicament contained in the capsule within the medicated module into the liquid during dispense of the medicaments the reservoir 222 has an integral flow distributor 232. This flow distributor also ensures efficient expulsion of the secondary-reservoir medicament from the system and greatly minimizes residual volume. One possible embodiment of the reservoir 222 and flow distributor 232 is illustrated in Figures 11 and 12. Preferably the reservoir and flow distributor are manufactured as a single part from materials that are compatible with the secondary-reservoir medicament, most preferably as a single molded piece. An example material would be that typically used to manufacture septa or pistons (bungs) found in multi-dose medicament cartridges, although any material that is compatible with the medicament during long term storage would be equally applicable. The flow distributor 232 is configured and positioned in reservoir 222 such that the secondary-reservoir medicament fills flow channels that are defined by the shape and location of one or more channels (not shown) inside the reservoir. The shape of the flow channels can be optimized for a plug flow of medicament by varying the dimensions of the flow distributor and/or channels. The cross-sectional area of the annulus formed between the flow distributor and the wall of the reservoir should be kept relatively small. The volume available to store the secondary-reservoir medicament would equal the internal volume of the reservoir minus the volume of the flow distributor. Therefore if the volume of the flow distributor is marginally smaller than the internal volume of the capsule, a small volume is left which the secondary-reservoir medicament occupies. Hence the scale of both the capsule and the flow distributor can be large while storing a small volume of medicament. Resultantly for small volumes of secondary-reservoir medicament (e.g. 50 micro liters) the reservoir can be of an acceptable size for handling, transport, manufacture, filling and assembly.

As described above, in one arrangement, Applicants' medicated module comprises a needle guard. However, in an alternative arrangement, a medicated module may be provided without a needle guard. As just one example, and as shown in Figure 13, a unique aspect of this medicated module 804 is the method of construction of output needle 803, part of which has an enlarged cross-section 805 to accommodate the volume of the fixed (single) non-user settable dose medicament 802. Preferably a hydroforming or a swaging process will be utilized to form the enlarged cross-section 805 of the needle 803. Both tips of the needle are preferably not enlarged which is beneficial because it helps minimize both the physical and mental/emotional trauma associated with insertion of larger bore needles as well as minimizing the risk of compromising the sealing integrity of the septa of the liquid container (multiple piercing of this type of material with a relatively large gauge needle increases the risk of "coring" of the septum).

To minimize the residual volume of the secondary-reservoir medicament that might remain in the medicated module or sub-assembly 804 at the end of the dispense operation caused by recirculation, the enlarged section 805 should be designed with fluid flow characterizing models. Preferably, the design of medicated module 804 should ensure that at least about 80% of the secondary-reservoir medicament is expelled through the distal end of needle 803, most preferably at least about 90% should be expelled. Ideally, displacement of the primary-reservoir liquid 801 into the proximal end 806 of needle 803 will displace the secondary-reservoir medicament 802 without substantial mixing of the two medicaments. Preferably this is accomplished by minimizing the diametric increase and careful design of the transition from the small cross sections of the needle 803 to the enlarged cross section 805. One alternative is to have the assembly/filling process set up so as to ensure that a "plug" of gas (e.g. air or an inert gas such as nitrogen) is present in the section 806 of the needle (above the enlarged section 805) this may act to ensure that the primary-reservoir liquid and the secondary-reservoir medicament are kept separate from each other thereby help ensure sequential delivery by action of a virtual piston created by the plug of air. This plug may additionally help ensure that there is no opportunity for the liquid and secondary-reservoir medicament to mix prior to injection (i.e. if the medicated module is left in the attached position for an extended period of time prior to the injection action being undertaken.

Attachment of the medicated module 804 to a drug delivery device, such as the device illustrated in Figure 1, causes the engagement needle 806 located in the module to penetrate the septum 808 of the drug delivery device 807, such as the syringe illustrated in Figure 1. Once the engagement needle has passed through the septum of the syringe, fluid connection is made between the primary-reservoir liquid 801 and the output needle 803. Dispense of the medicaments is then achieved by subcutaneously injecting the medicaments via activation of a plunger 30 on device 807 via a dose button 65.

Preferably the medicated module is provided by a drug manufacturer as a stand-alone and separate device that is sealed to preserve sterility. The sterile seal of the module is preferably designed to be opened automatically, e.g. by cutting, tearing or peeling, when the medicated module is advanced or attached to the drug delivery device by the user. Features such as angled surfaces on the end of the injection device or features inside the module may assist this opening of the seal.

The medicated module of our invention should be designed to operate in conjunction with a multiple use injection device. The injection device could be a reusable or disposable device. By disposable device it is meant an injection device that is obtained from the manufacturer preloaded with medicament and cannot be reloaded with new medicament after the initial medicament is exhausted.

Exemplary embodiments of the present invention have been described. Those skilled in the art will understand, however, that changes and modifications may be made to these embodiments without departing from the true scope and spirit of the present invention, which is defined by the claims.

### List of references

- 1: drug delivery system
- 5: primary device (i.e., syringe)
- 10: dosing mechanism
- 15: syringe body
- 20: syringe body distal end
- 22: syringe septum
- 25: syringe body proximal end
- 30: plunger
- 35: plunger distal end
- 40: plunger proximal end
- 45: piston
- 50: threaded end
- 55: exclusive attachment
- 60: primary-reservoir liquid (e.g., medicament)
- 65: plunger dosing button
- 70: primary reservoir
- 80: drug delivery device
- 85: distal end of drug delivery device
- 90: coupling mechanism
- 92a: first attachment means
- 92b: second attachment means
- 100: channels
- 120: engagement arms
- 130: distal needle
- 140: medicated module
- 150: proximal needle
- 160a: top septum / membrane / seal
- 160b: bottom septum/ membrane / seal
- 180: attachment means / connector
- 190: connection means/ attachment means
- 200: housing
- 202a,202b: keeps
- 210: hole
- 220: drive tooth
- 222: reservoir
- 230: track
- 232: flow distribution
- 240: path
- 240a: transition point
- 250: path
- 250a: transition point
- 255: shoulder cap
- 260: path
- 260a: transition point
- 260b: axial stop
- 270: legs
- 290: path
- 310: capsule
- 320: pocket stop
- 330: planar surface
- 390: path/directional arrow
- 400: radial stand off
- 420: guard
- 460: bypass
- 480: spring/biasing member
- 500: distal end of drug delivery device (syringe)
- 510: upper hub
- 520: bypass housing
- 530: lower hub
- 540: window
- 560: retention snap
- 650: lower stand off pocket
- 660: upper stand off pocket
- 700: lock out boss
- 710: lock out feature
- 732: upper stand off pocket stop
- 801: primary-reservoir liquid (e.g., medicament)
- 802: secondary-reservoir medicament
- 803: output needle
- 804: medicated module
- 805: enlarged cross section
- 806: needle proximal end
- 807: drug delivery device
- 808: drug delivery device septum

## Claims

1. A drug delivery system (1) to deliver at least one medicament operable through a drug delivery device (5; 80; 807) and a single dispense interface (140), comprising,
a. a housing (200) defining a primary reservoir (70), the primary reservoir (70) containing a liquid;
b. a dosing mechanism (10) operably connected to the primary reservoir (70);
c. a secondary reservoir (22) for a medicament comprising at least one drug agent configured for fluid communication to the single dispense interface (140);
d. and a single dispense (140) interface configured for fluid communication with the primary reservoir (70) and the secondary reservoir (222);
wherein a single activation of the dosing mechanism (10) causes liquid from the primary reservoir (70) and a non-user settable dose of the medicament to be expelled through the single dispense interface (140).

2. The system (1) of claim 1 wherein the drug delivery device (5; 80; 807) comprises a syringe.

3. The system (1) of claim 2 wherein the syringe comprises a prefilled syringe.

4. The system (1) of any preceding claim where a single operation of the dosing mechanism (10) causes a dose of the liquid from the primary reservoir (70) to be expelled through the single dispense interface (140) after at least substantially all of the non-user settable dose of the medicament has been expelled through the single dispense interface (140).

5. The system (1) of any preceding claim where the primary reservoir (70) contains multiple doses of the liquid.

6. The system (1) of any preceding claim where the secondary reservoir (222) contains multiple doses of the medicament.

7. The system (1) of any preceding claim where the secondary reservoir (222) and the single dispense interface (140) are contained in a medicated module (140) that is disposable and replaceable with a replacement module, wherein the replacement module comprises a new secondary reservoir of medicament and a new single dispense interface.

8. The system (1) of claim 7 where the medicated module (140) contains a needle guard (420).

9. The system (1) of claim 8 where the needle guard (420) is operably connected to the secondary reservoir (222).

10. The system (1) of any preceding claim wherein the drug delivery device (5; 80; 807) comprises a coupling mechanism (190) for releasably coupling the secondary reservoir (222) to the drug delivery device (5; 80; 807).

11. The system (1) of any preceding claim, wherein the liquid is selected from the group consisting of a solution, a solvent, and a second medicament.

12. The system (1) of any preceding claim wherein the secondary reservoir (220) comprises a medicated module (140) attachable to the drug delivery device (5; 80; 807), the medicated module comprising,
e. an outer housing (200) having an inner surface, a proximal end and a distal end, where the proximal end has an upper hub (510) holding a first double-ended needle cannula (803, 806) and a connector (190) configured for attachment to a drug delivery device;
f. a bypass housing (520) having an outer surface and slidably engaged with an upper radial stand off on the inner surface of the housing (520);
g. a reservoir within the bypass housing (520) comprising a single dose of the medicament;
h. a guard (420) having an internal proximal face and a drive tooth on an inner surface, where the drive tooth is slidably engaged with a track on the outer surface of the bypass housing;
i. a lower hub slidably engaged with the outer surface of the bypass housing (520) and slidably engaged with the inner surface of the guard (420); and
j. a biasing member (480) engaged between the internal proximal face of the guard and with the lower hub.

13. The system of (1) claim 12 where the lower hub holds a second double-ended needle cannula (803, 806).

14. The system (1) of claim 13 where the reservoir is a single molded component having an internal cavity with an integral flow distributor (232).

15. A method of dispensing at least one medicament, comprising, in combination, the steps of
a. providing a drug delivery device (5; 80; 807) comprising,
i. a housing (20) containing a single dose setter (10) operably connected to a primary reservoir (70), wherein the primary reservoir holds a liquid;
ii. a dose button (65) operably connected to the primary reservoir (70); and
iii. a single dispense interface (140) configured for fluid communication with the primary reservoir;
b. providing a secondary reservoir (220) configured for fluid communication to the single dispense interface (140), wherein the secondary reservoir (220) holds a medicament containing at least one drug agent, wherein a single activation of the dose button (65) causes liquid from the primary reservoir (70) and a non-user settable dose of the medicament from the secondary reservoir to be expelled through the single dispense interface (140).
